(19) Europäisches Patentamt
European Patent Office
Office européen des brevets

(11) **EP 4 349 243 A1**

(12) **EUROPEAN PATENT APPLICATION**
published in accordance with Art. 153(4) EPC

(43) Date of publication:
**10.04.2024 Bulletin 2024/15**

(21) Application number: **22811335.3**

(22) Date of filing: **24.05.2022**

(51) International Patent Classification (IPC):
**A61B 3/10** (2006.01)

(52) Cooperative Patent Classification (CPC):
**A61B 3/10**

(86) International application number:
**PCT/JP2022/021291**

(87) International publication number:
**WO 2022/250065 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.05.2021 JP 2021088766**

(71) Applicant: **Tomey Corporation
Nagoya-shi, Aichi 451-0051 (JP)**

(72) Inventors:
• **TOTANI, Kota
Nagoya-shi, Aichi 451-0051 (JP)**
• **SUGIYAMA, Satoshi
Nagoya-shi, Aichi 451-0051 (JP)**

(74) Representative: **Winter, Brandl - Partnerschaft
mbB
Alois-Steinecker-Straße 22
85354 Freising (DE)**

(54) **OPTICAL TOMOGRAM IMAGING DEVICE**

(57) An optical coherence tomographic device may include: an image capturing unit configured to capture n tomographic images from a capturing range which is set within a subject eye by executing a capturing process that scans the capturing range with light, the n being an integer of 2 or more; a generator configured to generate an evaluation index for evaluating an image quality of a tomographic image; and a display unit configured to display the evaluation index generated by the generator. The generator may be configured to generate the evaluation index for each of the n tomographic images. The display unit may be configured to simultaneously display the evaluation indexes of the n tomographic images on one screen.

FIG. 10

**Description**

TECHNICAL FIELD

The art disclosed herein relates to an optical coherence tomographic device.

BACKGROUND ART

An optical coherence tomographic device for obtaining a tomographic image of a subject eye is in development. The optical coherence tomographic device includes a measurement optical system that irradiates light from a light source onto a subject eye and guides reflected light therefrom and a reference optical system that generates reference light using light from the light source. Upon measurement, a tomographic image of the subject eye is generated from interference light obtained by combining the reflected light (measurement light) guided by the measurement optical system and the reference light generated by the reference optical system. When the tomographic image of the subject eye is not captured appropriately, the tomographic image of the subject eye needs to be recaptured. Due to this, in such an optical coherence tomographic device, an evaluation index indicating whether the tomographic image has been captured appropriately may be displayed on a display unit. For example, Japanese Patent Application Publication No. 2013-9798 describes, as an example of such an evaluation index, a QI (Quality Index) being an image quality evaluation index obtained from a histogram related to brightness values of an image.

SUMMARY OF INVENTION

TECHNICAL PROBLEM

In the optical coherence tomographic device of Japanese Patent Application Publication No. 2013-9798, whether the tomographic image has been captured appropriately or not is presented to an examiner by displaying the evaluation index. If the tomographic image was not captured appropriately then it needs to be recaptured, thus a subject must stay until the examiner confirms that the tomographic image has been captured appropriately. In the optical coherence tomographic device of Japanese Patent Application Publication No. 2013-9798, since the evaluation index is displayed for each tomographic image, the examiner must check the evaluation index for each of the plurality of tomographic images generated in one capturing session. Due to this, a time required for the examiner to check the evaluation indexes for all of the tomographic images becomes long, and a time during which the subject must stay for the capturing session was thereby lengthened.

The description herein discloses an art configured to reduce burden on a subject in capturing tomographic images.

SOLUTION TO TECHNICAL PROBLEM

An optical coherence tomographic device disclosed herein may comprise: an image capturing unit configured to capture n tomographic images from a capturing range which is set within a subject eye by executing a capturing process that scans the capturing range with light, the n being an integer of 2 or more; a generator configured to generate an evaluation index for evaluating an image quality of a tomographic image; and a display unit configured to display the evaluation index generated by the generator. The generator may be configured to generate the evaluation index for each of the n tomographic images. The display unit may be configured to simultaneously display the evaluation indexes of the n tomographic images on one screen.

In the above optical coherence tomographic device, by displaying the evaluation indexes of the plurality of (n) tomographic images obtained by executing the capturing process simultaneously on one screen, an examiner can check every evaluation result on the plurality of tomographic images captured in the capturing process within a short period of time. Due to this, the evaluation result does not have to be checked for each tomographic image at a time, and the time required for checking all the evaluation results for all of the tomographic images can be shortened.

BRIEF DESCRIPTION OF DRAWINGS

FIG. 1 shows a schematic configuration of optical systems of an optical coherence tomographic device according to an embodiment.
FIG. 2 is a block diagram showing a control system of the optical coherence tomographic device according to the embodiment.

FIG. 3 is a block diagram showing a configuration of a sampling trigger/clock generator.

FIG. 4 is a flowchart showing an example of a process of displaying a preview screen after having captured tomographic images of a subject eye.

FIG. 5 is a diagram for explaining an equation for calculating a QI (Quality Index).

FIG. 6 is a flowchart showing an example of a process of calculating a maximum brightness.

FIG. 7 is a diagram for explaining about a process of decimating A-scan information used in calculating the maximum brightness.

FIG. 8 is a diagram for explaining about a process of limiting a depthwise range of the A-scan information used in calculating the maximum brightness, where (a) shows a case where an image was captured with the subject eye in a suitable position and (b) shows a case where an image was captured with the subject eye being displaced.

FIG. 9 is a diagram showing QI evaluation results and evaluation results of a state of fixation, where (a) shows a case where the subject eye is in a suitable position, (b) shows a case where the subject eye blinked, and (c) shows a case where the subject eye is in a displaced position.

FIG. 10 is a diagram showing an example of a preview screen displayed on a monitor.

FIG. 11 is a diagram showing an example of the preview screen and a concise report displayed on the monitor.

DESCRIPTION OF EMBODIMENTS

Some of the features characteristic to below-described embodiments will herein be listed. It should be noted that the respective technical elements are independent of one another, and are useful solely or in combinations. The combinations thereof are not limited to those described in the claims as originally filed.

(Feature 1) In the optical coherence tomographic device disclosed herein, the evaluation index may comprise at least one of a brightness evaluation index and a fixation evaluation index, the brightness evaluation index being for evaluating the image quality based on a brightness of the tomographic image, and the fixation evaluation index being for evaluating a fixation state of the subject eye. According to such a configuration, when the brightness evaluation index is used, aspects such as appropriateness of alignment and focus, as well as presence of blinking of the subject eye can be evaluated. Further, by using the fixation evaluation index, aspects such as fixation not being appropriately achieved due to involuntary eye movement during fixation and presence of blinking of the subject eye can be evaluated. As such, by including at least one of the brightness evaluation index and the fixation evaluation index as the evaluation index, the image quality of the tomographic images can suitably be evaluated.

(Feature 2) In the optical coherence tomographic device disclosed herein, the generator may be configured to select m tomographic image(s) from the n tomographic images and further generate an examination report indicating an examination result obtained from the selected tomographic image(s), the m being a natural number smaller than the n. The display unit may be configured to further display the generated examination report. According to such a configuration, by displaying the examination report, whether the capturing session for the desired examination has been performed appropriately can be checked. Further, in generating the examination report, only m tomographic image(s) selected from n tomographic images are used rather than using all of the n tomographic images. Due to this, as compared to a case of generating the examination report from the n tomographic images, the concise examination report generated from the m tomographic images can be generated within a shorter period of time. As such, by generating the examination report as the concise examination report, whether the capturing session has been appropriately performed can be checked while shortening the time for generating the examination report.

EMBODIMENT

Hereinafter, an optical coherence tomographic device according to the present embodiment will be described. The optical coherence tomographic device according to the present embodiment is a polarization-sensitive OCT (PS-OCT) that is capable of capturing polarization characteristics of a subject to be examined by a Fourier domain method of a wavelength sweeping type using a wavelength sweeping light source (swept-source optical coherence tomography: SS-OCT).

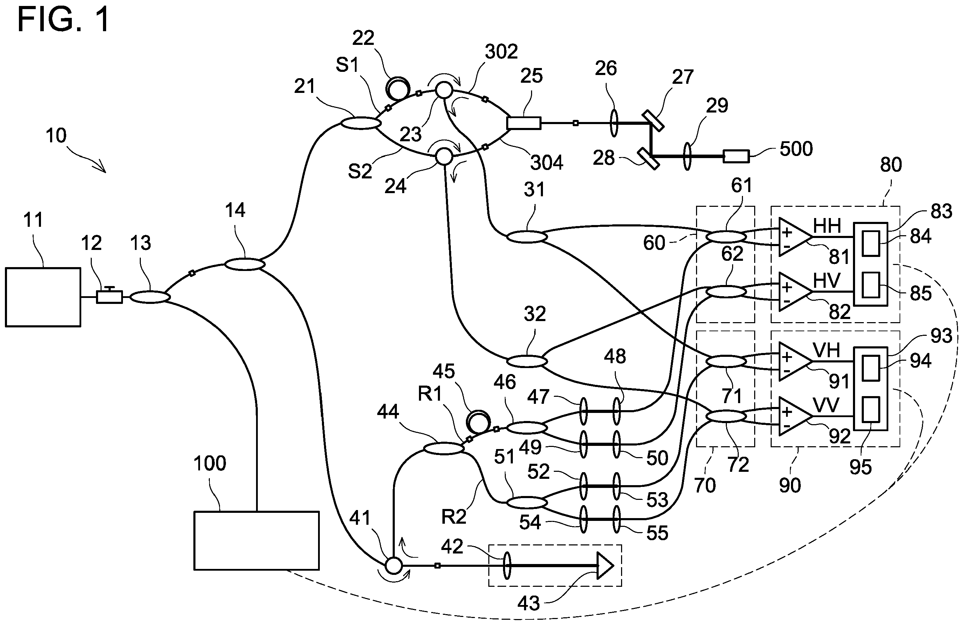

As illustrated in FIG. 1, the optical coherence tomographic device according to the present embodiment comprises a light source 11; a measurement light generator (21 to 29, 31, 32) that generates measurement light from light outputted from the light source 11; a reference light generator (41 to 46, 51) that generates reference light from the light outputted from the light source 11; interference light generators 60, 70 that combine reflected light from a subject eye 500 generated in the measurement light generator with the reference light generated in the reference light generator to generate interference light; and interference light detectors 80, 90 that detect the interference light generated in the interference light generators 60, 70.

(Light Source)

The light source 11 is a light source of a wavelength sweeping type, and the wavelength (wavenumber) of output light varies within a predetermined cycle. Since the wavelength of light with which the subject eye 500 is irradiated varies (swept), an intensity distribution of light reflected from respective portions of the subject eye 500 along a depth direction can be obtained by subjecting signals obtained from interference light, which is a combination of the reflected light from the subject eye 500 and the reference light, to Fourier analysis.

A polarization control device 12 and a fiber coupler 13 are connected to the light source 11, and a PMFC (polarization maintaining fiber coupler) 14 and a sampling trigger/clock generator 100 are connected to the fiber coupler 13. Therefore, the light outputted from the light source 11 is inputted to the PMFC 14 and the sampling trigger/clock generator 100 through the polarization control device 12 and the fiber coupler 13. The sampling trigger/clock generator 100 generates a sampling trigger and a sampling clock for each of signal processors 83 and 93 (which will be described later) by using the light from the light source 11.

(Measurement Light Generator)

The measurement light generator (21 to 29, 31, 32) comprises a PMFC 21 connected to the PMFC 14; two measurement light paths S1 and S2 branching off from the PMFC 21; a polarization beam combiner/splitter 25 connecting the two measurement light paths S1 and S2; a collimator lens 26 connected to the polarization beam combiner/splitter 25; galvanometer mirrors 27 and 28; and a lens 29. An optical path length difference generator 22 and a circulator 23 are disposed on the measurement light path S1. Only a circulator 24 is disposed on the measurement light path S2. Therefore, an optical path length difference ∆L between the measurement light path S1 and the measurement light path S2 is generated by the optical path length difference generator 22. The optical path length difference ∆L may be set to be longer than a depthwise measurement range of the subject eye 500. This prevents interference light with different optical path length differences from overlapping each other. As the optical path length difference generator 22, for example, an optical fiber may be used or an optical system such as a mirror, a prism, etc. may be used. In the present embodiment, a PM fiber with a length of one meter is used as the optical path length difference generator 22. The measurement light generator further comprises PMFCs 31, 32. The PMFC 31 is connected to the circulator 23. The PMFC 32 is connected to the circulator 24.

One of light (i.e., measurement light) split by the PMFC 14 is inputted to the measurement light generator (21 to 29, 31, 32). The PMFC 21 splits the measurement light inputted from the PMFC 14 into first measurement light and second measurement light. The first measurement light split by the PMFC 21 is guided to the measurement light path S1, and the second measurement light split by the PMFC 21 is guided to the measurement light path S2. The first measurement light guided to the measurement light path S1 is inputted to the polarization beam combiner/splitter 25 through the optical path length difference generator 22 and the circulator 23. The second measurement light guided to the measurement light path S2 is inputted to the polarization beam combiner/splitter 25 through the circulator 24. A PM fiber 304 is connected to the polarization beam combiner/splitter 25 such that the PM fiber 304 is circumferentially turned by 90 degrees relative to a PM fiber 302. For this reason, the second measurement light inputted to the polarization beam combiner/splitter 25 has a polarization component orthogonal to the first measurement light. Since the optical path length difference generator 22 is disposed on the measurement light path S1, the first measurement light is delayed relative to the second measurement light by a distance corresponding to the optical path length difference generator 22 (that is, the optical path length difference ∆L is generated). The polarization beam combiner/splitter 25 superimposes the inputted first measurement light and second measurement light. The light outputted from the polarization beam combiner/splitter 25 (superimposed light of the first measurement light and the second measurement light) passes through the collimator lens 26, the galvanometer mirrors 27 and 28, and the lens 29 and is then irradiated onto the subject eye 500. The light irradiated onto the subject eye 500 is scanned along an x-y direction by the galvanometer mirrors 27 and 28.

The light irradiated onto the subject eye 500 is reflected by the subject eye 500. The reflected light by the subject eye 500 scatters at the surface of the subject eye 500 and the inside thereof. The reflected light from the subject eye 500 passes through, in the reverse order to the incidence path, the lens 29, the galvanometer mirrors 28, 27, and the collimator lens 26, and is then inputted to the polarization beam combiner/splitter 25. The polarization beam combiner/splitter 25 splits the inputted reflected light into two polarization components that are orthogonal to each other. These are termed horizontal polarization reflected light (horizontal polarization component) and vertical polarization reflected light (vertical polarization component), for convenience sake. The horizontal polarization reflected light is guided to the measurement light path S1, and the vertical polarization reflected light is guided to the measurement light path S2.

The optical path of the horizontal polarization reflected light is changed by the circulator 23, and the horizontal polarization reflected light is inputted to the PMFC 31. The PMFC 31 splits the inputted horizontal polarization reflected light so that it is inputted to each of PMFCs 61, 71. Therefore, the horizontal polarization reflected light inputted to each of the PMFCs 61, 71 contains a reflected light component based on the first measurement light and a reflected light component based on the second measurement light. The optical path of the vertical polarization reflected light is changed by the circulator 24, and the vertical polarization reflected light is inputted to the PMFC 32. The PMFC 32 splits the inputted vertical polarization reflected light so that it is inputted to each of PMFCs 62, 72. Therefore, the vertical polarization reflected light inputted to each of the PMFCs 62, 72 contains a reflected light component based on the first measurement light and a reflected light component based on the second measurement light.

(Reference Light Generator)

The reference light generator (41 to 46, 51) comprises a circulator 41 connected to the PMFC 14; a reference delay line (42, 43) connected to the circulator 41; a PMFC 44 connected to the circulator 41; two reference light paths R1 and R2 branching off from the PMFC 44; a PMFC 46 connected to the reference light path R1; and a PMFC 51 connected to the reference light path R2. An optical path length difference generator 45 is disposed on the reference light path R1. No optical path length difference generator is disposed on the reference light path R2. Therefore, an optical path length difference ΔL' between the reference light path R1 and the reference light path R2 is generated by the optical path length difference generator 45. For example, an optical fiber is used as the optical path length difference generator 45. The optical path length difference ΔL' of the optical path length difference generator 45 may be the same as the optical path length difference ΔL of the optical path length difference generator 22. If the optical path length differences ΔL and ΔL' are the same, depthwise positions of a plurality of interference light (described later) in the subject eye 500 coincide with each other. That is, it is unnecessary to align a plurality of acquired tomographic images.

The other of light split by the PMFC 14 (i.e., reference light) is inputted to the reference light generator (41 to 46, 51). The reference light inputted from the PMFC 14 is inputted to the reference delay line (42, 43) through the circulator 41. The reference delay line (42, 43) includes a collimator lens 42 and a reference mirror 43. The reference light inputted to the reference delay line (42, 43) is irradiated to the reference mirror 43 through the collimator lens 42. The reference light reflected by the reference mirror 43 is inputted to the circulator 41 through the collimator lens 42. The reference mirror 43 is movable in directions to approach and separate from the collimator lens 42. In the present embodiment, the position of the reference mirror 43 is adjusted before the start of measurement so that a signal from the subject eye 500 will be within an OCT depthwise measurable range.

The optical path of the reference light reflected by the reference mirror 43 is changed by the circulator 41, and the reference light reflected by the reference mirror 43 is inputted to the PMFC 44. The PMFC 44 splits the inputted reference light into first reference light and second reference light. The first reference light is guided to the reference light path R1, and the second reference light is guided to the reference light path R2. The first reference light is inputted to the PMFC 46 through the optical path length difference generator 45. The reference light inputted to the PMFC 46 is split into first split reference light and second split reference light. The first split reference light is inputted to the PMFC 61 through a collimator lens 47 and a lens 48. The second split reference light is inputted to the PMFC 62 through a collimator lens 49 and a lens 50. The second reference light is inputted to the PMFC 51 and then is split into third split reference light and fourth split reference light. The third split reference light is inputted to the PMFC 71 through a collimator lens 52 and a lens 53. The fourth split reference light is inputted to the PMFC 72 through a collimator lens 54 and a lens 55.

(Interference Light Generator)

The interference light generators 60, 70 include a first interference light generator 60 and a second interference light generator 70. The first interference light generator 60 includes the PMFCs 61 and 62. As described, the horizontal polarization reflected light from the measurement light generator and the first split reference light (light having the optical path length difference ΔL') from the reference light generator are inputted to the PMFC 61. Here, the horizontal polarization reflected light contains a reflected light component (light having the optical path length difference ΔL) based on the first measurement light and a reflected light component (light that does not have the optical path length difference ΔL) based on the second measurement light. Therefore, in the PMFC 61, the first split reference light is combined with the reflected light component (light having the optical path length difference ΔL) based on the first measurement light which is among the horizontal polarization reflected light, as a result of which first interference light (horizontal polarization component) is generated.

The vertical polarization reflected light from the measurement light generator and the second split reference light (light having the optical path length difference ΔL') from the reference light generator are inputted to the PMFC 62. Here, the vertical polarization reflected light contains a reflected light component (light having the optical path length difference ΔL) based on the first measurement light and a reflected light component (light that does not have the optical path length difference ΔL) based on the second measurement light. Therefore, in the PMFC 62, the second split reference light is combined with the reflected light component (light having the optical path length difference ΔL) based on the first measurement light which is among the vertical polarization reflected light, as a result of which second interference light (vertical polarization component) is generated.

The second interference light generator 70 includes the PMFCs 71 and 72. As described, the horizontal polarization reflected light from the measurement light generator and the third split reference light (light that does not have the optical path length difference ΔL') from the reference light generator are inputted to the PMFC 71. Therefore, in the PMFC 71, the third split reference light is combined with a reflected light component (light that does not have the optical path length difference ΔL) based on the second measurement light which is among the horizontal polarization reflected light, as a result of which third interference light (horizontal polarization component) is generated.

The vertical polarization reflected light from the measurement light generator and the fourth split reference light (light that does not have the optical path length difference ΔL') from the reference light generator are inputted to the PMFC 72. Therefore, in the PMFC 72, the fourth split reference light is combined with the reflected light component (light that does not have the optical path length difference ΔL) based on the second measurement light which is among the vertical polarization reflected light, as a result of which fourth interference light (vertical polarization component) is generated. The first interference light and the second interference light correspond to the measurement light that has passed through the measurement light path S1, and the third interference light and the fourth interference light correspond to the measurement light that has passed through the measurement light path S2.

(Interference Light Detectors)

The interference light detectors 80, 90 include a first interference light detector 80 configured to detect the interference light (the first interference light and the second interference light) generated in the first interference light generator 60, and a second interference light detector 90 configured to detect the interference light (the third interference light and the fourth interference light) generated in the second interference light generator 70.

The first interference light detector 80 comprises balanced light detectors 81 and 82 (which may simply be termed detectors 81, 82 hereinbelow), and a signal processor 83 connected to the detectors 81 and 82. The PMFC 61 is connected to the detector 81, and the signal processor 83 is connected to an output terminal of the detector 81. The PMFC 61 splits the first interference light into two interference light that have phases different from each other by 180 degrees, and inputs the two interference light to the detector 81. The detector 81 performs differential amplification processing and noise reduction processing to the two interference light having phases different from each other by 180 degrees inputted from the PMFC 61 so as to convert them to an electric signal (first interference signal), and outputs the first interference signal to the signal processor 83. That is, the first interference signal is an interference signal HH between the reference light and the horizontal polarization reflected light from the subject eye 500 based on the horizontal polarization measurement light. Similarly, the PMFC 62 is connected to the detector 82, and the signal processor 83 is connected to an output terminal of the detector 82. The PMFC 62 splits the second interference light into two interference light that have phases different from each other by 180 degrees, and inputs the two interference light to the detector 82. The detector 82 performs differential amplification processing and noise reduction processing to the two interference light having phases different from each other by 180 degrees so as to convert them to an electric signal (second interference signal), and outputs the second interference signal to the signal processor 83. That is, the second interference signal is an interference signal HV between the reference light and the vertical polarization reflected light from the subject eye 500 based on the horizontal polarization measurement light.

The signal processor 83 comprises a first signal processing unit 84 to which the first interference signal is inputted, and a second signal processing unit 85 to which the second interference signal is inputted. The first signal processing unit 84 is configured to sample the first interference signal based on a sampling trigger and a sampling clock inputted to the signal processor 83 from the sampling trigger/clock generator 100. The second signal processing unit 85 is configured to sample the second interference signal based on the sampling trigger and the sampling clock inputted to the signal processor 83 from the sampling trigger/clock generator 100. The first and second interference signals sampled in the first signal processing unit 84 and the second signal processing unit 85 are inputted to a processor 202 (which will be described later). A known data acquisition device (a so-called DAQ) may be used as the signal processor 83.

Similar to the first interference light detector 80, the second interference light detector 90 comprises balanced light detectors 91 and 92 (which may simply be termed detectors 91, 92 hereinbelow), and the signal processor 93 connected to the detectors 91 and 92. The PMFC 71 is connected to the detector 91, and the signal processor 93 is connected to an output terminal of the detector 91. The PMFC 71 splits the third interference light into two interference light that have phases different from each other by 180 degrees, and inputs the two interference light to the detector 91. The detector 91 performs differential amplification processing and noise reduction processing to the two interference light having phases different from each other by 180 degrees so as to convert them to an electric signal (third interference signal), and outputs the third interference signal to the signal processor 93. That is, the third interference signal is an interference signal VH between the reference light and the horizontal polarization reflected light from the subject eye 500 based on the vertical polarization measurement light. Similarly, the PMFC 72 is connected to the detector 92, and the signal processor 93 is connected to an output terminal of the detector 92. The PMFC 72 splits the fourth interference light into two interference light that have phases different from each other by 180 degrees, and inputs the two interference light to the detector 92. The detector 92 performs differential amplification processing and noise reduction processing to the two interference light having phases different from each other by 180 degrees so as to convert them to an electric signal (fourth interference signal), and outputs the fourth interference signal to the signal processor 93. That is, the fourth interference signal is an interference signal VV between the reference light and the vertical polarization reflected light from the subject eye 500 based on the vertical polarization measurement light.

The signal processor 93 comprises a third signal processing unit 94 to which the third interference signal is inputted, and a fourth signal processing unit 95 to which the fourth interference signal is inputted. The third signal processing unit 94 is configured to sample the third interference signal based on a sampling trigger and a sampling clock inputted to the signal processor 93 from the sampling trigger/clock generator 100. The fourth signal processing unit 95 is configured to sample the fourth interference signal based on the sampling trigger and the sampling clock inputted to the signal processor 93 from the sampling trigger/clock generator 100. The third and fourth interference signals sampled in the third signal processing unit 94 and the fourth signal processing unit 95 are inputted to the processor 202 (which will be described later). A known data acquisition device (a so-called DAQ) may also be used as the signal processor 93. According to the above configuration, it is possible to acquire the interference signals indicative of four polarization characteristics of the subject eye 500. In the present embodiment, the signal processors 83, 93, each of which comprises two signal processing units, are used, however, different configurations may be employed. For example, one signal processor comprising four signal processing units may be used, or four signal processors each comprising one signal processing unit may be used.

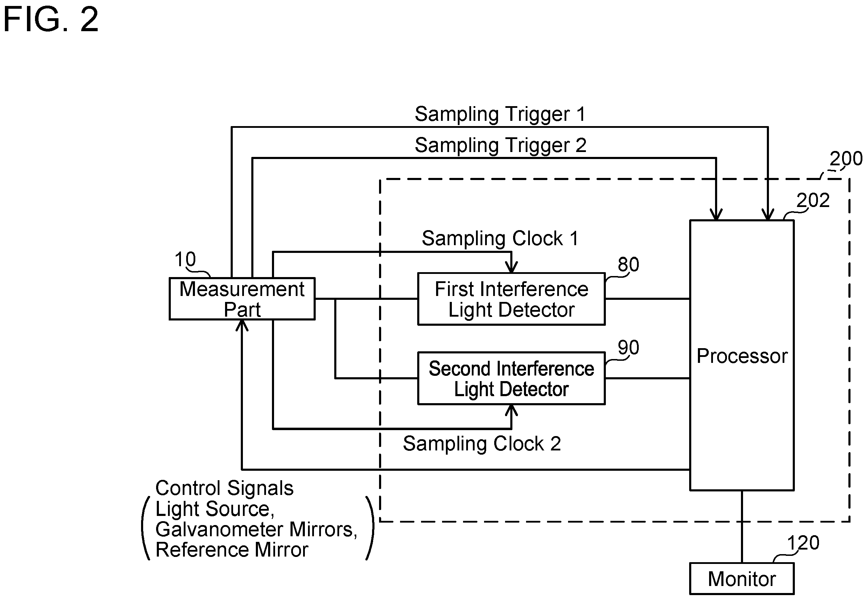

Next, the configuration of a control system of the optical coherence tomographic device according to the present embodiment will be described. As illustrated in FIG. 2, the optical coherence tomographic device is controlled by a calculation unit 200. The calculation unit 200 comprises the processor 202, the first interference light detector 80, and the second interference light detector 90. The first interference light detector 80, the second interference light detector 90, and the processor 202 are connected to a measurement unit 10. The processor 202 is configured to output a control signal to the measurement unit 10 to move an incidence position of the measurement light to the subject eye 500 by driving the galvanometer mirrors 27 and 28. The first interference light detector 80 acquires first sampling data with respect to the interference signals (the interference signal HH and the interference signal HV) inputted from the measurement unit 10 based on a sampling clock 1 inputted from the measurement unit 10 and by using a sampling trigger 1 as a trigger, and outputs the first sampling data to the processor 202. The processor 202 performs calculation processing such as Fourier transform, etc. to the first sampling data to generate an HH tomographic image and an HV tomographic image. The second interference light detector 90 acquires second sampling data with respect to the interference signals (the interference signal VH and the interference signal VV) inputted from the measurement unit 10 based on a sampling clock 2 inputted from the measurement unit 10 and by using a sampling trigger 2 as a trigger, and outputs the second sampling data to the processor 202. The processor 202 performs calculation processing such as Fourier transform, etc. to the second sampling data to generate a VH tomographic image and a VV tomographic image. The HH tomographic image, the VH tomographic image, the HV tomographic image, and the VV tomographic image are tomographic images at the same position. Thus, the processor 202 can create tomographic images with four polarization characteristics (HH, HV, VH, VV) that represent a Jones matrix of the subject eye 500.

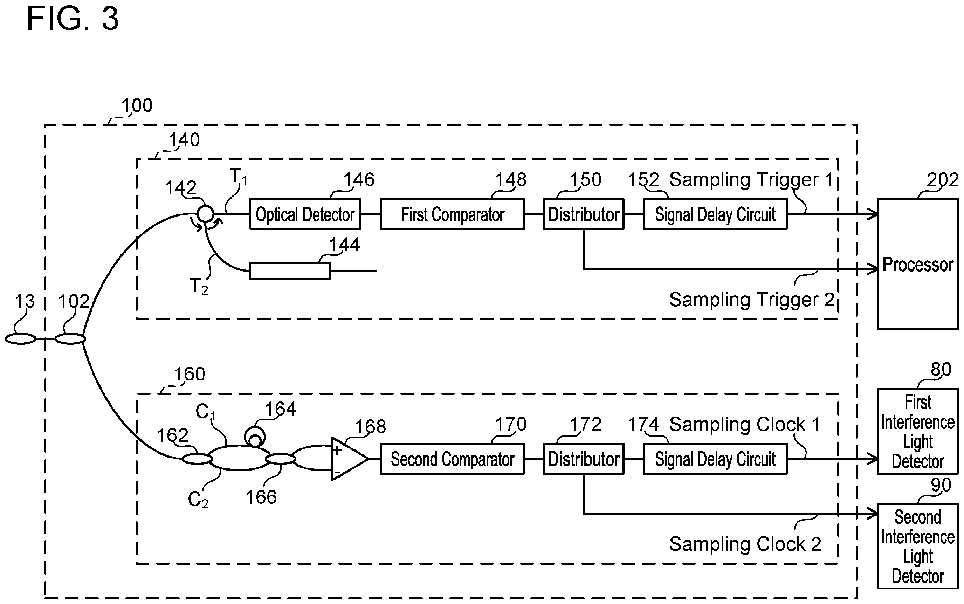

As illustrated in FIG. 3, the sampling trigger/clock generator 100 comprises a fiber coupler 102, a sampling trigger generator (140 to 152), and a sampling clock generator (160 to 172). The light from the light source 11 is inputted, through the fiber coupler 13 and the fiber coupler 102, to each of the sampling trigger generator 140 and the sampling clock generator 160.

(Sampling Trigger Generator)

The sampling trigger generator 140 may generate a sampling trigger by using, for example, an FBG (fiber bragg grating) 144. As illustrated in FIG. 3, the FBG 144 reflects only a component of the light inputted from the light source 11 that has a specific wavelength, thereby generating a sampling trigger. The generated sampling trigger is inputted to a distributor 150. The distributor 150 distributes the sampling trigger into the sampling trigger 1 and the sampling trigger 2. The sampling trigger 1 is inputted, through a signal delay circuit 152, to the processor 202. The sampling trigger 2 is directly inputted to the processor 202. The sampling trigger 1 is a trigger signal for the interference signals (the first interference signal and the second interference signal) inputted from the first interference light detector 80 to the processor 202. The sampling trigger 2 is a trigger signal for the interference signals (the third interference signal and the fourth interference signal) inputted from the second interference light detector 90 to the processor 202. The signal delay circuit 152 is designed such that the sampling trigger 1 is delayed relative to the sampling trigger 2 by a time corresponding to the optical path length difference $\Delta L$ of the optical path length difference generator 22. This makes it possible to make a frequency at which the sampling of the interference signals inputted from the first interference light detector 80 is started equal to a frequency at which the sampling of the interference signals inputted from the second interference light detector 90 is started. Only the sampling trigger 1 may be generated. Since the optical path length difference $\Delta L$ is known, the sampling of the interference signals inputted from the second interference light detector 90 may be started such that the start time is delayed from the sampling trigger 1 by a time corresponding to the optical path length difference $\Delta L$.

(Sampling Clock Generator)

The sampling clock generator may be configured of a Mach-Zehnder interferometer, for example. As illustrated in FIG. 3, the sampling clock generator generates a sampling clock with the same frequency by using the Mach-Zehnder interferometer. The sampling clock generated by the Mach-Zehnder interferometer is inputted to a distributor 172. The distributor 172 distributes the sampling clock into the sampling clock 1 and the sampling clock 2. The sampling clock 1 is inputted, through a signal delay circuit 174, to the first interference light detector 80. The sampling clock 2 is directly inputted to the second interference light detector 90. The signal delay circuit 174 is designed to cause a delay by a time corresponding to the optical path length difference $\Delta L$ of the optical path length difference generator 22. This makes it possible to sample interference light with the delay corresponding to the optical path length difference generator 22 at the same timing. Thus, positional misalignment among a plurality of acquired tomographic images can be prevented. In the present embodiment, a Mach-Zehnder interferometer is used to generate the sampling clocks. Alternatively, a Michelson interferometer or an electric circuit may be used to generate the sampling clocks. Alternatively, the sampling clocks may be generated by using a light source including a sampling clock generator.

Further, the optical coherence tomographic device according to the present embodiment comprises an SLO (Scanning Laser Ophthalmoscope) optical system (not shown) configured to obtain a front image of the subject eye 500. Those used in publicly known ophthalmic devices can be used as this SLO optical system, thus explanation on the detailed configuration thereof will be omitted.

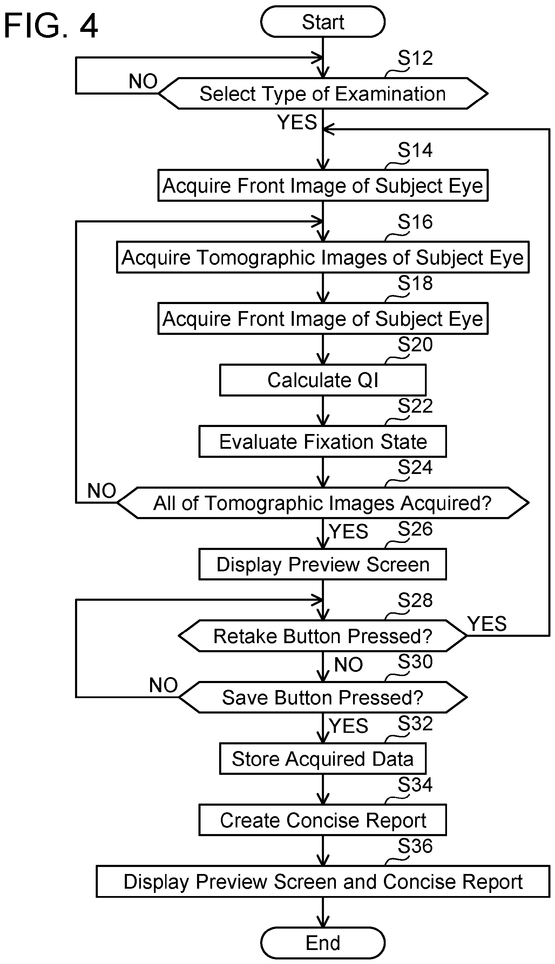

Next, a process of displaying a preview screen after having captured tomographic images of the subject eye 500 will be explained with reference to FIG. 4. The preview screen is a screen used for the examiner to determine whether a capturing session for the subject eye 500 has been performed appropriately. In the optical coherence tomographic device according to the present embodiment, a plurality of tomographic images is captured within a set range of the subject eye 500 in order to obtain data related to a desired examination of the subject eye 500 (such as an examination requested by a doctor). If the subject eye 500 moves for example by involuntary eye movement during fixation or blinks while capturing the plurality of tomographic images, all of those tomographic images cannot be captured appropriately. Due to this, evaluation index(es) and/or an image (preview screen) indicating whether each tomographic image has been captured appropriately are displayed on the monitor 120 after the capturing session of the subject eye 500. The examiner determines whether the respective tomographic images are captured appropriately by using the preview screen, and decides whether the subject eye 500 is to be recaptured.

As shown in FIG. 4, firstly the processor 202 determines whether a type of examination has been selected (S12). As described above, the optical coherence tomographic device according to the present embodiment is a polarization-sensitive optical coherence tomographic device, thus a tomographic image captured by irradiating vertical waves to the subject eye 500 and a tomographic image captured by irradiating horizontal waves to the subject eye 500 can be obtained simultaneously. By using these two types of tomographic images, the processor 202 can not only generate a tomographic image indicating tissues in the subject eye 500 by scattering intensity (a so-called 'regular' tomographic image) but also generate a tomographic image indicating entropy in the subject eye 500, a tomographic image indicating double refraction in the subject eye 500, a tomographic image indicating extending direction(s) of fibers in the subject eye 500, a tomographic image indicating a blood flow in the subject eye 500, and the like. The processor 202 uses these multiple types of tomographic images to generate an examination report corresponding to the type of examination.

In starting the examination on the subject eye 500, the examiner uses an input means such as a mouse (not shown) to select a desired examination from a plurality of examinations displayed on the monitor 120. Then, when the work to select the type of examination is completed, the examiner instructs completion of the selection work. For example, the examiner instructs the completion of the selection work by using the input means to press an "OK" button displayed on the monitor 120. The processor 202 waits until the completion of the selection work is instructed by the examiner as above (NO to step S12).

When the completion of the selection work is instructed (YES to step S12), the processor 202 obtains a front image of the subject eye 500 (S14). Specifically, the examiner performs positioning of the optical coherence tomographic device relative to the subject eye 500 by operating an operation member such as a joystick that is not shown. That is, the processor 202 drives a position adjusting mechanism that is not shown in accordance with the examiner's operation on the operation member. Due to this, the position of the optical coherence tomographic device in xy directions (vertical and horizontal directions) and the position thereof in a z direction (advancing and retracting direction) relative to the subject eye 500 are adjusted. When the positioning of the optical coherence tomographic device is performed, the processor 202 captures the front image of the subject eye 500 using the SLO optical system. The captured front image is stored in a memory (not shown) of the processor 202. The front image obtained hereof is used as a reference image upon evaluating the state of fixation in the following process (more specifically, in the process of step S22). Hereinbelow, the front image obtained in step S14 may be termed "reference front image".

Next, the processor 202 obtains a tomographic image of the subject eye 500 (S16). In the present embodiment, the tomographic image is obtained by irradiating light to the subject eye 500 using a raster scanning scheme. Due to this, a tomographic image of a retina of the subject eye 500 in a square region with a set position as a center is obtained. The capturing method of the tomographic image of the retina of the subject eye 500 is not limited to the raster scanning scheme. So long as the tomographic image of the retina of the subject eye 500 can be captured over an entirety of a desired region, the image may be captured with a radial scanning scheme, for example. Further, the processor 202 obtains the front image of the subject eye 500 (S18). This front image is obtained substantially simultaneously as the tomographic image obtained in step S16.

Then, the processor 202 calculates QI (Quality Index) of the tomographic image obtained in step S16 (S20). The QIis a brightness evaluation index for evaluating the image quality based on the brightness of the tomographic image. For example, if the focus is not set correctly or the positional relationship between the subject eye 500 and the optical coherence tomographic device is not appropriate, the brightness of the tomographic image may become low and the tomographic image may not be captured appropriately. Further, if there is an image with low brightness among the captured tomographic images, a variation in the brightness occurs among the tomographic images, and the desired examination report may not be generated with high accuracy. As such, the respective tomographic images are evaluated based on the brightness. Here, an example of a QI calculation method will be described. The QI is calculated using an equation indicated in Math 1 below.

[Math 1]

$$QI = \mathrm{round}(\ (\mathrm{max}\text{-}\mathrm{noise})\text{-}\mathrm{cut})/(\mathrm{range}\text{-}\mathrm{cut}) * 10)$$

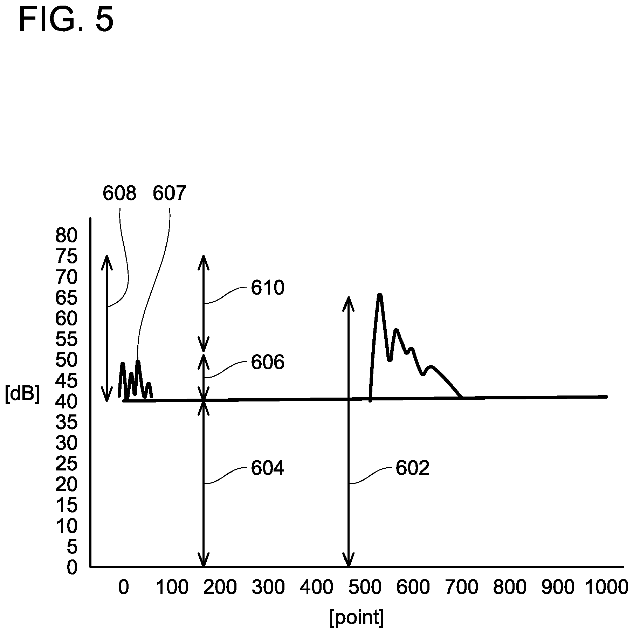

A function of the equation represented in Math 1 above will be described with reference to FIG. 5. "Round" indicates a function for rounding to an integer. "Max" indicates a maximum brightness 602 of the interference signal obtained from a specific position of the fundus of the subject eye 500, and in FIG. 5, it is for example about 65dB. A calculation method of the maximum brightness 602 will be described later. "Noise" indicates the brightness 604 of a portion where there is no image of the subject eye 500 (that is, a portion where scattered light is not generated) (noise floor brightness), and in FIG. 5, it is set for example to about 40dB or less. "Cut" indicates a brightness 606 that does not contribute to image evaluation (cutoff brightness), and it is for example set in a range of about 10dB from a noise floor brightness 604 (which is about 40dB in FIG. 5) (the range being about 40 to 50dB in FIG. 5). As such, a signal 607 indicating the brightness within the range of the cutoff brightness 606 is determined as a signal not necessary for the QI calculation. "Range" indicates a brightness range 608 to which 256 shades are allocated, and it is for example set in a range of about 35dB from the noise floor brightness 604 (which is about 40dB in FIG. 5) (the range being about 40 to 75dB in FIG. 5). Further, "10" in the equation represented in Math 1 as above indicates that the QI is evaluated in scales of 10. The QI is an index that evaluates the brightness in the range 610, which excluded the cutoff brightness 606 from the brightness range 608, in the scales of 10.

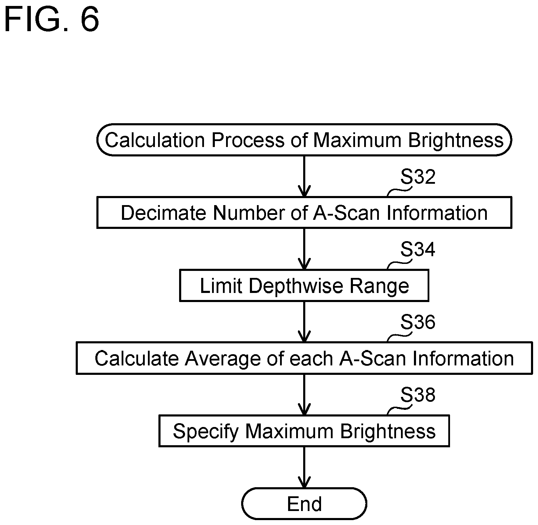

Here, the calculation method of the maximum brightness 602 will be described. In the present embodiment, the maximum brightness 602 is calculated using the following method in order to shorten the calculation time for the maximum brightness 602. As shown in FIG. 6, firstly the processor 202 selects A-scan information used for calculating the maximum brightness 602 from among a plurality of A-scan information constituting the tomographic image (S32). That is, when the subject eye 500 is to be measured, tomographic information indicating the relationship between a depthwise position along a measurement optical axis and a signal intensity (so-called A-scan information) is obtained from the interference light. To obtain the tomographic image of the subject eye 500, the measurement light is scanned to obtain a plurality of A-scan information, and the tomographic image of the subject eye 500 is generated using such plurality of A-scan information. In step S32, the plurality of A-scan information constituting the tomographic image is decimated to reduce the A-scan information used for calculating the maximum brightness 602.

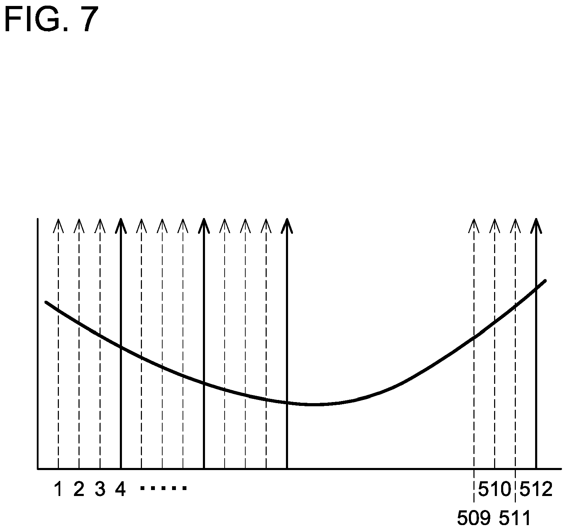

More specific explanation will be given with reference to FIG. 7. FIG. 7 schematically shows a tomographic image, where a curve shows a surface of the retina of the subject eye 500, and arrows indicate A-scan information constituting this tomographic image. In FIG. 7, the tomographic image is assumed as being constituted of 512 sets of A-scan information. In step S32, the A-scan information is decimated so that only one A-scan information in every four A-scan information among the 512 A-scan information is used. That is, for consecutive A-scan information, one in every four A-scan information is selected (arrows shown in solid lines in FIG. 7), and the three A-scan information located in between (arrows shown in dotted lines in FIG. 7) are not selected. By doing so, in the example of FIG. 7, 128 sets of A-scan information are selected from among the 512 sets of A-scan information. Due to this, the A-scan information used in the calculation of the maximum brightness 602 is reduced to 1/4, and calculation cost (calculation load) is reduced to 1/4. On the other hand, one in every four A-scan information scanned consecutively is selected at a regular pattern, and even in comparing with the case of calculating the maximum brightness 602 using all the A-scan information, the calculation accuracy of the maximum brightness 602 does not significantly decrease. As such, by decimating the A-scan information, the calculation cost of the maximum brightness 602 can be reduced while hardly decreasing the calculation accuracy of the maximum brightness 602.

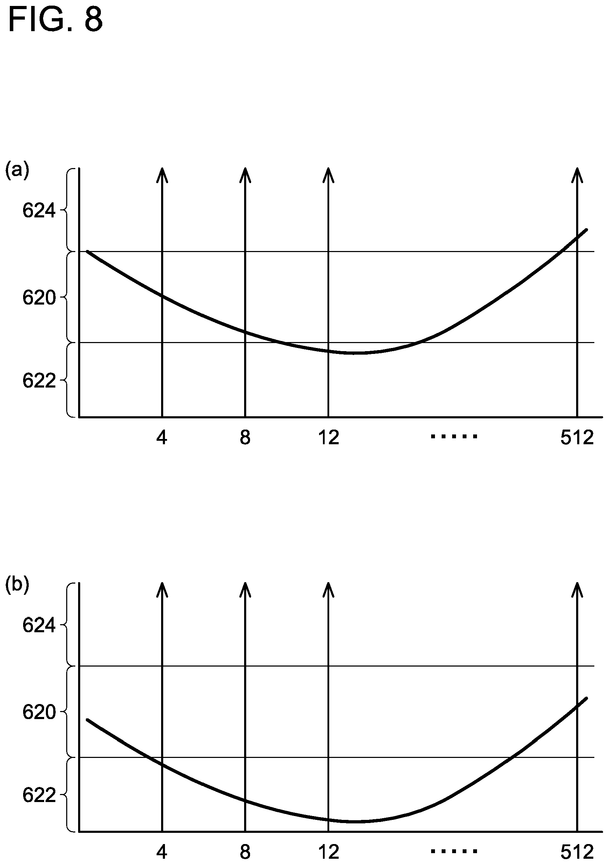

Next, the processor 202 limits the depthwise range of the A-scan information used in the calculation of the maximum brightness 602 to a preset range (S34). Specifically, as shown in FIG. 8(a), when the subject eye 500 is located at an appropriate position, the depthwise range of the A-scan information used in the calculation of the maximum brightness 602 is limited to a range 620 around a center of the depthwise range in which the subject eye 500 is captured. If the tomographic image is captured with the subject eye 500 located at the appropriate position, majority of the portions of the tomographic image of the subject eye 500 (tissues on which scattered light is generated) is included in the range 620, and not much of the tomographic image of the subject eye 500 (tissues on which scattered light is generated) is included in a range 622 that is deeper than the range 620 and a range 624 that is shallower than the range 620. As such, by limiting the depthwise range of the A-scan information to the aforementioned range, the maximum brightness 602 in the depthwise range 620 around the center with the subject eye 500 located at the appropriate position is calculated.

As shown in FIG. 8(b), if the subject eye 500 is captured while being located at a position displaced from the appropriate position, a capturing range of the subject eye 500 (portions where scattered light is generated) is displaced in the depthwise direction (in FIG. 8(b), it is displaced toward the range 622 that is deeper than the range 620), and the portion captured outside the range 620 increases. If the depthwise range is limited as the range 620 near the center for the case with the subject eye 500 located at the appropriate position and the subject eye 500 is captured while being located at the position displaced from the appropriate position, the capturing range of the subject eye 500 (portions where scattered light is generated) is hardly included in the range 620. Due to this, for the tomographic image captured in the state where the subject eye 500 is located at a position displaced from the appropriate position, the calculated maximum brightness becomes small, and the QI evaluation becomes low. Further, the tomographic image captured in the state where the subject eye 500 is displaced is positionally displaced in the depthwise direction from other tomographic image(s) captured at the appropriate position, thus becomes an inappropriate image. There would be no problem even if the QI is low for such an inappropriate tomographic image. Thus, by limiting the depthwise range of the A-scan information, the calculation cost (calculation load) of the maximum brightness 602 can be reduced without causing an issue on the QI evaluation.

Next, the processor 202 calculates an average for the respective A-scan information with the number of the A-scan information having been decimated in step S32 as above in the range limited in the depthwise direction in step S34 (S36). The average calculated hereof is set as the brightness value of each A-scan information.

Lastly, the processor 202 specifies the maximum brightness 602 from the brightness values (average) of the respective A-scan information calculated in step S36 (S38). Specifically, the processor 202 specifies a largest brightness value (average) among the brightness values (average) of the A-scan information calculated in step S36 as the maximum brightness 602. The processor 202 substitutes the maximum brightness 602 calculated as above to the equation represented in Math 1 as above to calculate the QI. Due to this, the QI can be calculated for the acquired tomographic image.

As shown in FIG. 4, when the QI is calculated, the processor 202 evaluates the state of fixation (S22). The evaluation on the state of fixation is performed by comparing the front image of the subject eye 500 acquired in step S18 (that is, the front image acquired substantially at the same time as the tomographic image of the subject eye 500 acquired in step S16) with the reference front image acquired in step S14.

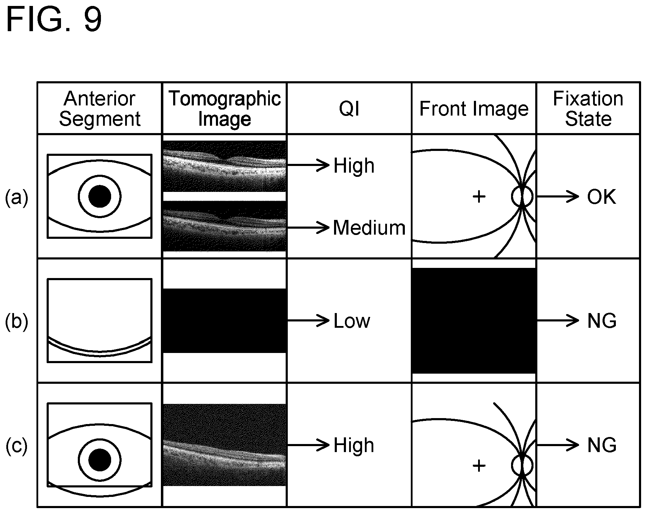

An evaluation method of the state of fixation will be described with reference to FIG. 9. FIG. 9(a) shows a case where the subject eye 500 does not move due to involuntary eye movement during fixation or the like, and thus is in the appropriate position. When the subject eye 500 is in the appropriate position, the front image acquired in step S18 substantially matches the reference front image. In this case, the processor 202 determines that the state of fixation is appropriate. As shown in FIG. 9(b), the front image is not captured in step S18 if the eye is blinking. In this case, the processor 202 determines "inappropriate" as the evaluation of the state of fixation. As shown in FIG. 9(c), if the subject eye 500 is moving due to involuntary eye movement during fixation, the front image captured in step S18 does not match the reference front image. For example, in FIG. 9(c), the subject eye 500 is captured at a lower position than in the case where it is at the appropriate position (case of FIG. 9(a)), thus it does not match the position of the subject eye 500 in the reference front image. In this case, the processor 202 determines that the state of fixation is inappropriate.

In the evaluation of the QI, as described above, the depthwise range of the A-scan information is limited for calculating the maximum brightness 602 in step S34, thus the QI may be possibly determined low when the capturing position of the subject eye 500 is significantly displaced. However, for example, if the displacement in the capturing position of the subject eye 500 is small, the maximum brightness 602 is calculated with a large value. Further, if the subject eye 500 is displaced only horizontally (that is, displaced in a plane perpendicular to the optical axis), the maximum brightness 602 is calculated with a large value. In such cases, as shown in FIG. 9(c), the QI may be determined high.

On the other hand, the evaluation of the state of fixation is performed by comparing the front image acquired in step S18 with the reference front image. Due to this, as shown in FIG. 9(c), the determination that the subject eye 500 was captured in a displaced state can be made with high accuracy. As above, by evaluating not only the QI but also the state of fixation, an inappropriate state that cannot be determined by sole evaluation of the QI (that is, the subject eye 500 was captured in the state of being displaced horizontally) can be specified.

As shown in FIG. 4, the processor 202 then determines whether all the tomographic images from the set capturing range have been acquired (S24). If all the tomographic images have not been acquired (NO to step S24), it returns to step S16 and repeats the processes of steps S16 to S24.

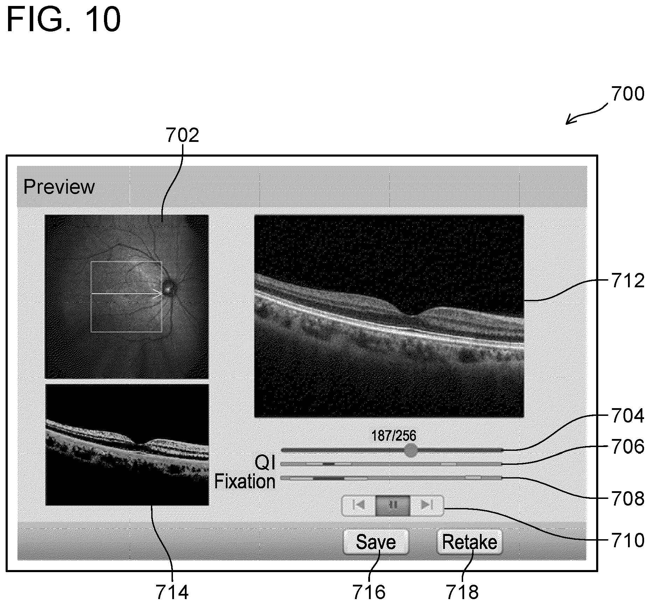

Then, the processor 202 causes the monitor 120 to display a preview screen 700 including the evaluation result for the QI and the evaluation result for the state of fixation (S26). As shown in FIG. 10, the preview screen 700 includes a front image 702 of the subject eye 500, evaluation result for the QI (which is bar 706 in FIG. 10), the evaluation result for the state of fixation (which is bar 708 in FIG. 10), a tomographic image 712 of the subject eye 500, a tomographic image 714 indicating the entropy in the subject eye 500, a "Save" button 716, and a "Retake" button 718. The front image 702 of the subject eye 500 is the front image acquired in step S14 (that is, the reference front image), and the tomographic image capturing range (range surrounded by a square line) is indicated in the front image 702. The tomographic image 712 is a tomographic image that indicates the tissues in the subject eye 500 by scattering intensity (so-called "regular" tomographic image), and is the one selected from among the plurality of tomographic images. The tomographic image 714 indicating the entropy in the subject eye 500 is of the center in the capturing range. By displaying the tomographic image 714 indicating the entropy in the subject eye 500 as a representative, confirmation can be made on whether the capturing session for generating the tomographic image indicating the entropy in the subject eye 500 has been appropriately performed or not. The "Save" button 716 and the "Retake" button 718 are configured capable of being pressed by the examiner using the input means such as a mouse (not shown).

The evaluation result for the QI is determined based on the QI calculated in step S20. For example, the processor 202 categorizes in to three classes, being a case where the QI is low (such as 1 to 4), a case where the QI is medium (such as 5 to 7), and a case where the QI is high (such as 8 to 10). Then, the evaluation results of all the tomographic images are simultaneously displayed on one screen so that the examiner can see the evaluation results of all the tomographic images at a glance. For example, in the example of FIG. 10, the QI evaluation result is displayed by the bar 706 (hereinbelow may be termed "QI bar 706"). With the QI bar 706, the three categories are indicated with different colors. For example, the QI bar 706 indicates the evaluation result for the case of high QI with green, the evaluation result for the case of medium QI with yellow, and the evaluation result for the case of low QI with red. In the present embodiment, 256 tomographic images are captured, and the respective tomographic images are numbered 1 to 256 along the cross section. The QI bar 706 displays the QI evaluation results of the tomographic images corresponding to the $1^{st}$ to $256^{th}$ tomographic images with different colors arranged along the left-right direction. Due to this, the QI evaluation results for all of the tomographic images can be checked at a glance.

The evaluation results of the state of fixation are displayed based on the evaluation of the state of fixation in step S22. The evaluation results of the state of fixation are similarly displayed by the bar 708 (hereinbelow may be termed "fixation bar 708"). For example, the fixation bar 708 indicates the evaluation results of the case with appropriate state of fixation (that is, the front image acquired in step S18 substantially matches the reference front image) with green, and indicates the evaluation results of the case with inappropriate state of fixation (that is, the front image acquired in step S18 does not match the reference front image) with red. Further, if the divergence between the front image acquired in step S18 and the reference front image is trivial, the evaluation results may be indicated with yellow, for it is moderately appropriate. The fixation bar 708 is displayed below the QI bar 706. The fixation bar 708 displays the evaluation results on the state of fixation of the tomographic images corresponding to the $1^{st}$ to $256^{th}$ tomographic images with different colors arranged along the left-right direction. Due to this, the evaluation results on the state of fixation for all of the tomographic images can be checked at a glance.

A bar 704 indicating the respective tomographic images (hereinbelow may be termed image sliding bar 704) is displayed parallel to the QI bar 706 and the fixation bar 708 at a position higher than those bars. Further, the monitor 120 displays a selection button 710 for selecting each of the tomographic images. By using the selection button 710, the image sliding bar 704 can be moved to the left or right to select a tomographic image. The monitor 120 displays the selected tomographic image 712. The QI bar 706 and the fixation bar 708 display the evaluation results of the tomographic images at positions corresponding to the respective tomographic images in the image sliding bar 704. Due to this, the examiner can easily specify the tomographic image determined as inappropriate in the QI bar 706 and the fixation bar 708. Then, the examiner can display the tomographic image determined as inappropriate in the QI bar 706 and the fixation bar 708, and individually check whether this tomographic image was appropriately captured or not. As above, by using the QI bar 706 and the fixation bar 708, the tomographic image determined as inappropriate can easily be specified, thus the burden of the check work on the examiner can be decreased.

Next, the processor 202 determines whether the "Retake" button 718 was pressed or not (S28). The "Retake" button 718 can be pressed by the examiner using the input means such as a mouse (not shown). The examiner checks the preview screen 700 displayed in step S26, and when determining that the subject eye 500 was not captured appropriately, he/she presses the "Retake" button 718. When the "Retake" button 718 is pressed (YES to step S28), the processor 202 captures the subject eye 500 again. That is, it returns to step S14 and repeats the processes of steps S14 to S28.

On the other hand, if the "Retake" button 718 is not pressed (NO to step S28), the processor 202 determines whether the "Save" button 716 was pressed or not (S30). The "Save" button 716 can be pressed by the examiner using the input means such as a mouse (not shown). The examiner checks the preview screen 700 displayed in step S26, and when determining that the subject eye 500 was captured appropriately, he/she presses the "Save" button 716. When the "Save" button 716 is pressed (YES to step S30), the processor 202 stores the tomographic image of the subject eye 500 acquired in step S16 in the memory of the processor 202 (not shown) (S32). On the other hand, when the "Save" button 716 is not pressed (NO to step S30), it returns to step S28 and repeats the processes of steps S28 to S30 until either the "Retake" button 718 or the "Save" button 716 is pressed.

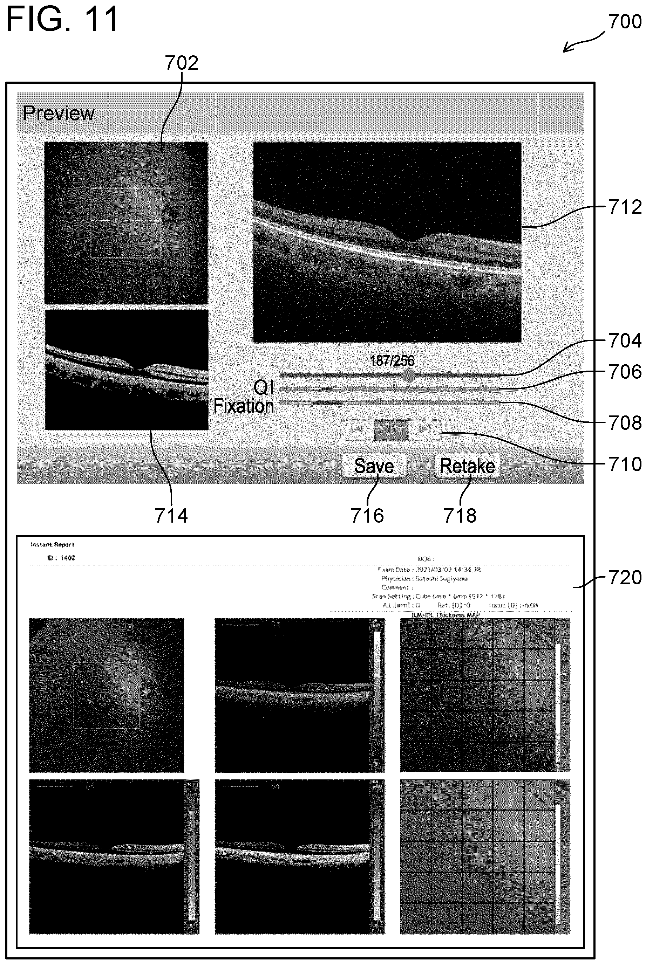

When the tomographic image of the subject eye 500 is saved, the processor 202 generates a concise report (S34). Here, the concise report refers to an examination report generated using a part of the entire set of tomographic images and corresponding to the desired examination selected in step S12. In the present embodiment, a total of 128 tomographic images of the subject eye 500 are acquired. The processor 202 selects five among the 128 tomographic images, and generates the examination report for the desired examination selected in step S12 as the concise report. When the concise report is generated, as shown in FIG. 11, the processor 202 displays a concise report 720 generated in step S34 on the monitor 120 along with the preview screen 700 displayed in step S26 (S36).

The concise report limits the number of tomographic images used in analysis, thus the time required for its generation process is shortened. The concise report uses only a part of the tomographic images in the analysis. As such, even if it is insufficient as examination report, the examiner can check the concise report so as to determine whether an affected site is captured as intended by referring to an image that cannot be displayed in the preview screen 700, such as polarized images and/or determine whether the subject eye 500 has been captured appropriately by checking the comparison result with the normal eye database. Here, the examination report generated using all the tomographic images (in the present embodiment, 128 tomographic images) is automatically generated after the process shown in FIG. 4 is completed and the capturing session for the subject eye 500 has been completed.

In the present embodiment, the examiner checks display of the QI evaluation results, the evaluation results on the state of fixation, and the concise report that are displayed on the monitor 120 and determines whether the capturing session was performed appropriately. In the present embodiment, by using the QI bar 706 and the fixation bar 708, the QI evaluation results and the evaluation results on the state of fixation for all the tomographic images are displayed simultaneously on one screen. Due to this, the time for determining whether the capturing session was appropriately performed based on the QI and the state of fixation can be shortened. Further, the concise report allows to determine whether the capturing session was appropriately performed so that the desired examination report can be generated. The concise report has a shorter generation time than the examination report generated using all the tomographic images. Due to this, the time for displaying the preview screen 700 can be shortened, and the time required from the start of the capturing session to the completion of the check using the preview screen 700 can be shortened.

Further when the examiner determines that the capturing session was not appropriately performed, the subject eye 500 is captured again. On the other hand, if the examiner determines that the capturing session was appropriately performed, the capturing session for the subject eye 500 is terminated. In the present embodiment, the time for determining whether the capturing session was appropriately performed can be shortened, thus the time during which the subject must stay for the capturing session of the subject eye 500 can be shortened.

In the present embodiment, the evaluation results for the QI and the evaluation results for the state of fixation are displayed using the bars, however, these are not limited to such configurations. So long as the evaluation results for the QI and/or the evaluation results for the state of fixation for all the tomographic images are displayed simultaneously on one screen, the evaluation results for the QI and/or the evaluation results for the state of fixation may for example be displayed using graphs, or may be displayed simultaneously on one screen as scores.

Further, in the present embodiment, the preview screen 700 is displayed and the concise report 720 is generated after having saved the tomographic images of the subject eye 500, however, it is not limited to such a configuration. For example, the concise report 720 may be generated before saving the tomographic images of the subject eye 500, and the preview screen 700 and the concise report 720 may be displayed on the monitor 120. In this case, the examiner can simultaneously check the preview screen 700 and the concise report 720 to determine whether the tomographic images of the subject eye 500 have been captured appropriately.

Further, in the present embodiment, the polarization-sensitive optical coherence tomographic device was used, however, it is not limited to such a configuration. The type of optical coherence tomography is not particularly limited, and it may for example be an optical coherence tomographic device that is not polarization sensitive.

Specific examples of the disclosure herein have been described in detail, however, these are mere exemplary indications and thus do not limit the scope of the claims. The art described in the claims includes modifications and variations of the specific examples presented above. Technical features described in the description and the drawings may technically be useful alone or in various combinations, and are not limited to the combinations as originally claimed. Further, the purpose of the examples illustrated by the present description or drawings is to satisfy multiple objectives simultaneously, and satisfying any one of those objectives gives technical utility to the present disclosure.

**Claims**

1. An optical coherence tomographic device comprising:

an image capturing unit configured to capture n tomographic images from a capturing range which is set within a subject eye by executing a capturing process that scans the capturing range with light, the n being an integer of 2 or more;
a generator configured to generate an evaluation index for evaluating an image quality of a tomographic image; and
a display unit configured to display the evaluation index generated by the generator,
wherein
the generator is configured to generate the evaluation index for each of the n tomographic images, and
the display unit is configured to simultaneously display the evaluation indexes of the n tomographic images on one screen.

2. The optical coherence tomographic device according to claim 1, wherein
the evaluation index comprises at least one of a brightness evaluation index and a fixation evaluation index, the brightness evaluation index being for evaluating the image quality based on a brightness of the tomographic image, and the fixation evaluation index being for evaluating a fixation state of the subject eye.

3. The optical coherence tomographic device according to claim 1 or 2, wherein

the generator is configured to select m tomographic image(s) from the n tomographic images and further generate an examination report indicating an examination result obtained from the selected tomographic image(s), the m being a natural number smaller than the n, and
the display unit is configured to further display the generated examination report.

FIG. 1

EP 4 349 243 A1

FIG. 2

Sampling Trigger 1
Sampling Trigger 2

200
202

Sampling Clock 1

10

Measurement
Part

First Interference
Light Detector  80

Second Interference
Light Detector  90

Processor

Sampling Clock 2

Control Signals
Light Source,
Galvanometer Mirrors,
Reference Mirror

120

Monitor

EP 4 349 243 A1

# FIG. 3

EP 4 349 243 A1

# FIG. 4

```
                    ( Start )
                        |
                        v
  ┌──────────────────> |           S12
  | NO <  Select Type of Examination  >
  |              YES |
  |                  v                S14
  |         [ Acquire Front Image of Subject Eye ]
  |                  |
  |  ┌────────────>  v                S16
  |  |      [ Acquire Tomographic Images of Subject Eye ]
  |  |               |                S18
  |  |               v
  |  |      [ Acquire Front Image of Subject Eye ]
  |  |               |                S20
  |  |               v
  |  |         [ Calculate QI ]
  |  |               |                S22
  |  |               v
  |  |      [ Evaluate Fixation State ]
  |  |               |                S24
  |  |   NO          v
  |  └────<  All of Tomographic Images Acquired?  >
  |                YES |              S26
  |                    v
  |         [ Display Preview Screen ]
  |                    |
  |  ┌─────────────>   v              S28
  |  |       <  Retake Button Pressed?  >  YES ──┐
  |  |               NO |              S30        |
  |  |   NO             v                         |
  |  └────<  Save Button Pressed?  >             |
  |                YES |              S32         |
  |                    v                          |
  |         [ Store Acquired Data ]               |
  |                    |              S34         |
  |                    v                          |
  |         [ Create Concise Report ]             |
  |                    |              S36         |
  |                    v                          |
  |  [ Display Preview Screen and Concise Report ]|
  |                    |                          |
  |                    v                          |
  |                 ( End )                        |
  └───────────────────────────────────────────────┘
```

# FIG. 5

# FIG. 6

```
┌──────────────────────────────────────────────────┐
│   Calculation Process of Maximum Brightness        │
└──────────────────────────────────────────────────┘
                         │
                         ▼           S32
┌──────────────────────────────────────────────────┐
│        Decimate Number of A-Scan Information        │
└──────────────────────────────────────────────────┘
                         │
                         ▼           S34
        ┌─────────────────────────────────┐
        │       Limit Depthwise Range       │
        └─────────────────────────────────┘
                         │
                         ▼           S36
┌──────────────────────────────────────────────────┐
│     Calculate Average of each A-Scan Information     │
└──────────────────────────────────────────────────┘
                         │
                         ▼           S38
        ┌─────────────────────────────────┐
        │      Specify Maximum Brightness    │
        └─────────────────────────────────┘
                         │
                         ▼
                   ┌───────────┐
                   │    End     │
                   └───────────┘
```

# FIG. 7

# FIG. 8

(a)

(b)

# FIG. 9

| | Anterior Segment | Tomographic Image | QI | Front Image | Fixation State |
|---|---|---|---|---|---|
| (a) | | | → High<br>→ Medium | + | → OK |
| (b) | | | → Low | | → NG |
| (c) | | | → High | + | → NG |

FIG. 10

700

702

Preview

712

187/256

704
706

QI
Fixation

708

710

Save   Retake

714        716      718

# FIG. 11

700

702

Preview

712

704
706

QI
Fixation

708

710

187/256

Save    Retake

714       716       718

720

Instant Report
ID : 1402                                          DOB :
                                    Exam Date : 2021/03/02 14:34:38
                                    Physician : Satoshi Sugiyama
                                    Comment :
                                    Scan Setting : Cube 6mm * 6mm (512 * 128)
                                    A.L.[mm] : 0    Ref. [D] :0    Focus [D] :-6.08
                                    ILM-IPL Thickness MAP

## INTERNATIONAL SEARCH REPORT

| International application No. |
| --- |
| **PCT/JP2022/021291** |

### A.   CLASSIFICATION OF SUBJECT MATTER

***A61B 3/10***(2006.01)i
FI:   A61B3/10 100

According to International Patent Classification (IPC) or to both national classification and IPC

### B.   FIELDS SEARCHED

Minimum documentation searched (classification system followed by classification symbols)

A61B3/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.   DOCUMENTS CONSIDERED TO BE RELEVANT

| Category* | Citation of document, with indication, where appropriate, of the relevant passages | Relevant to claim No. |
| --- | --- | --- |
| X | JP 2019-47842 A (CANON KK) 28 March 2019 (2019-03-28) | 1, 3 |
| | paragraphs [0012], [0086], [0104]-[0107], [0111], [0117]-[0118], [0125], fig. 8a, 8d, 8f | |
| Y | | 2 |
| X | JP 2014-83266 A (NIDEK CO LTD) 12 May 2014 (2014-05-12) | 1, 3 |
| | abstract, paragraphs [0052], [0124]-[0127], [0173]-[0176], [0211]-[0215], fig. 4 | |
| Y | | 2 |
| Y | JP 2013-9798 A (CANON KK) 17 January 2013 (2013-01-17) | 2 |
| | paragraphs [0004]-[0005] | |
| Y | JP 2014-226514 A (CANON KK) 08 December 2014 (2014-12-08) | 2 |
| | paragraphs [0070], [0110]-[0138] | |
| A | WO 2009/008125 A1 (OLYMPUS MEDICAL SYSTEMS CORP) 15 January 2009 (2009-01-15) | 1-3 |
| | abstract, paragraphs [0013]-[0062], fig. 1-7 | |

☑ Further documents are listed in the continuation of Box C.        ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 July 2022** | **16 August 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
|---|
| **PCT/JP2022/021291** |

**C.    DOCUMENTS CONSIDERED TO BE RELEVANT**

| Category* | Citation of document, with indication, where appropriate, of the relevant passages | Relevant to claim No. |
|---|---|---|
| A | JP 2016-529993 A (KONINKLIJKE PHILIPS N.V) 29 September 2016 (2016-09-29) abstract, paragraphs [0029], [0031]-[0032], fig. 2A-2D | 1-3 |

Form PCT/ISA/210 (second sheet) (January 2015)

INTERNATIONAL SEARCH REPORT
Information on patent family members

International application No.

**PCT/JP2022/021291**

| Patent document cited in search report | | | Publication date (day/month/year) | Patent family member(s) | | | Publication date (day/month/year) |
|---|---|---|---|---|---|---|---|
| JP | 2019-47842 | A | 28 March 2019 | US paragraphs [0024], [0097], [0115]-[0118], [0122], [0128]-[0129], [0136], fig. 8a, 8d, 8f CN | 2019/0069772 109464121 | A1 A | |
| JP | 2014-83266 | A | 12 May 2014 | US abstract, paragraphs [0090], [0162]-[0165], [0211]-[0214], [0249]-[0253], fig. 4 EP | 2014/0112562 2725508 | A1 A1 | |
| JP | 2013-9798 | A | 17 January 2013 | US paragraphs [0006]-[0007] | 2013/0003074 | A1 | |
| JP | 2014-226514 | A | 08 December 2014 | US paragraphs [0118], [0179]-[0225] EP CN KR | 2014/0347627 2807974 104173023 10-2014-0139438 | A1 A1 A A | |
| WO | 2009/008125 | A1 | 15 January 2009 | US abstract, paragraphs [0057]-[0111], fig. 1A-7 EP CN | 2010/0182412 2174578 101686799 | A1 A1 A | |
| JP | 2016-529993 | A | 29 September 2016 | US abstract, paragraphs [0037], [0039]-[0040], fig. 2A-2D WO CN | 2016/0192850 2015/022604 105451645 | A1 A2 A | |

Form PCT/ISA/210 (patent family annex) (January 2015)

**EP 4 349 243 A1**

## REFERENCES CITED IN THE DESCRIPTION

### Patent documents cited in the description

- JP 2013009798 A